# EUROPEAN PATENT APPLICATION

(11) **EP 4 108 195 A1**
(43) Date of publication of application: **28.12.2022**
(21) Application number: 20920335.5
(22) Date of filing: 19.02.2020
(51) Int. Cl.: A61B 17/86

(54) **IMPLANT**

(71) Applicant: Olympus Terumo Biomaterials Corp., Tokyo 151-0073 (JP)
(72) Inventor: ATARASHI, Masaki, Tokyo 151-0073 (JP); TAKAMI, Kimiaki, Tokyo 151-0073 (JP); KURODA, Koichi, Tokyo 151-0073 (JP)
(74) Representative: Schicker, Silvia
(86) International application number: PCT/JP2020/006385
(87) International publication number: WO 2021/166091

(57) **Abstract**

An implant (1) including an implant body (2) to be inserted into biological tissue, the implant body (2) having a hollow portion (2c) that penetrates the implant body (2), and an antibacterial property-imparting means (3) that imparts an antibacterial property to at least the hollow portion (2c) of the implant body (2).

## Description

### {Technical Field}

The present invention relates to an implant, in particular, to an implant having antibacterial properties.

### {Background Art}

Conventionally, in orthopedic surgery, osteosynthesis using a bone plate and a bone screw has been performed as a treatment method for a fracture or the like (for example, see PTL 1). As a clinical problem in osteosynthesis, infection accounts for a large proportion of cases, and there has been a demand for reducing the infection rate. For this reason, an implant having a surface subjected to antibacterial treatment is used (for example, see PTL 2).

### {Citation List}

### {Patent Literature}

{PTL 1} Japanese Unexamined Patent Application, Publication No. 2015-167779
{PTL 2} Publication of Japanese Patent No. 5590596

### {Summary of Invention}

### {Technical Problem}

There are some cases where a hollow screw is used as a bone screw in order to improve the surgical operability and reduce stress on a patient. After such a hollow implant is inserted into tissue inside a body, such as a bone, a space that communicates with outside of the body is formed in the interior of the tissue by means of a hollow portion of the implant. This space can be one of the main infection paths.

The present invention has been conceived in light of the abovementioned circumstances, and an object thereof is to provide a hollow implant capable of effectively suppressing infection.

### {Solution to Problem}

To achieve the abovementioned object, the present invention provides the following solutions.

An aspect of the present invention is an implant including: an implant body to be inserted into biological tissue, the implant body having a hollow portion that penetrates the implant body; and an antibacterial property-imparting means that imparts an antibacterial property to at least the hollow portion of the implant body.

In a state in which the implant body is inserted into tissue inside a living body, a space that communicates with outside of the body is formed in the interior of the tissue by means of the hollow portion. This space formed by the hollow portion can be one of the main infection paths. With this aspect, the antibacterial property-imparting means imparts an antibacterial property to the hollow portion. Furthermore, because the hollow portion does not come into direct contact with the tissue, the antibacterial property of the hollow portion can be enhanced without increasing the influence on fusion between the implant body and the tissue and the influence on cells around the implant body. Thus, it is possible to effectively suppress infection.

In the abovementioned aspect, the antibacterial property-imparting means may impart a higher level of the antibacterial property to the hollow portion as compared with an outer surface of the implant body.

In contrast to the antibacterial property of the hollow portion, the antibacterial property on the outer surface of the implant body that comes into direct contact with the tissue directly affect the cells and the fusion with the tissue. With this configuration, it is possible to effectively suppress the influence on the cells and the fusion with the tissue by reducing the antibacterial property on the outer surface.

In the abovementioned aspect, the outer surface of the implant body may not be subjected to antibacterial treatment.

With this configuration, it is possible to enhance the biocompatibility on the outer surface of the implant body that comes into direct contact with the tissue, thereby further suppressing the influence on the cells and the fusion with the tissue.

In the abovementioned aspect, the antibacterial property-imparting means may be an antibacterial layer that is formed by means of antibacterial treatment on a surface of the implant body, and the antibacterial layer may be formed on at least an inner surface of the hollow portion.

With this configuration, it is possible to impart the antibacterial property to the implant body itself.

In the abovementioned aspect, the antibacterial layer may be a silver layer.

Silver has high antibacterial properties and is also confirmed to have high biocompatibility. Therefore, by employing a silver layer as the antibacterial layer, it is possible to achieve both antibacterial properties and biocompatibility of the implant. In order to suppress the influence of the silver layer on the cells and the fusion with the tissue, the silver layer is preferably formed only on the inner surface of the hollow portion among the surfaces of the implant body.

In the abovementioned aspect, a film thickness of the silver layer is preferably equal to or more than 0.1 to equal to or less than 10 µm.

When the implant body is inserted into the biological tissue, there is a possibility that the silver layer may be peeled off as a result of a tool inserted into the hollow portion coming into contact with the silver layer on the inner surface of the hollow portion. By limiting the film thickness to 10 µm or less, it is possible to suppress the peeling amount of the silver layer. In addition, by setting the film thickness of the silver layer to 0.1 µm or more, it is possible to ensure sufficient antibacterial properties of the silver layer.

In the abovementioned aspect, the inner surface of the hollow portion may have a recessed portion that is recessed toward an outside of the implant body, and the antibacterial layer may be formed in the recessed portion.

The antibacterial layer in the recessed portion is unlikely to come into contact with the tool inside the hollow portion. Therefore, it is possible to prevent peeling of the antibacterial layer due to contact with the tool.

In the abovementioned aspect, the antibacterial property-imparting means may be an antibacterial member that is inserted into the hollow portion.

With this configuration, it is possible to impart an antibacterial property to the hollow portion with a simple operation merely by inserting the antibacterial member into the hollow portion. In addition, as the implant body, it is possible to use an implant body in which the hollow portion does not have antibacterial properties.

In the abovementioned aspect, the implant may be a bone screw including, as the implant body, a screw body that is screwed into a bone, and the hollow portion may penetrate the screw body in a direction along a longitudinal axis of the screw body.

### {Advantageous Effects of Invention}

The present invention affords an advantage in that it is possible to effectively suppress infection.

### {Brief Description of Drawings}

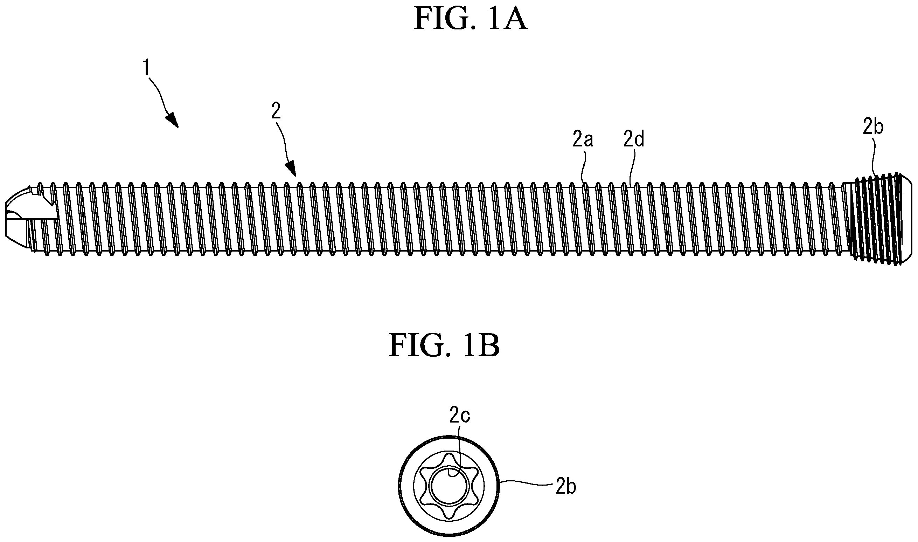

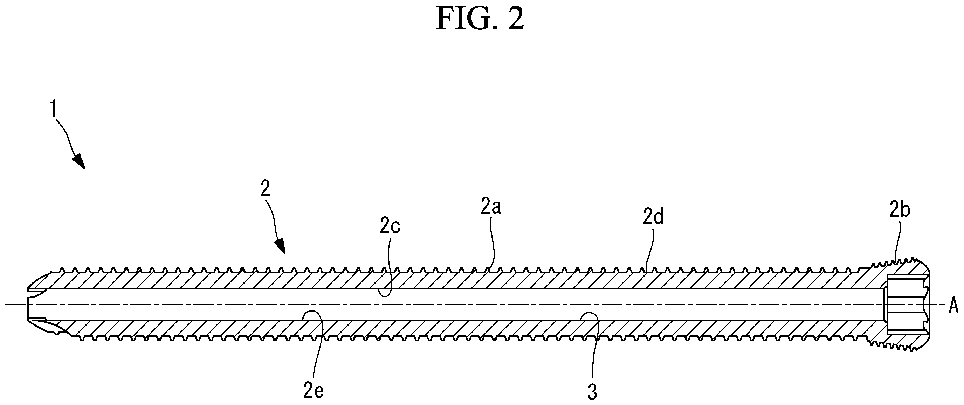

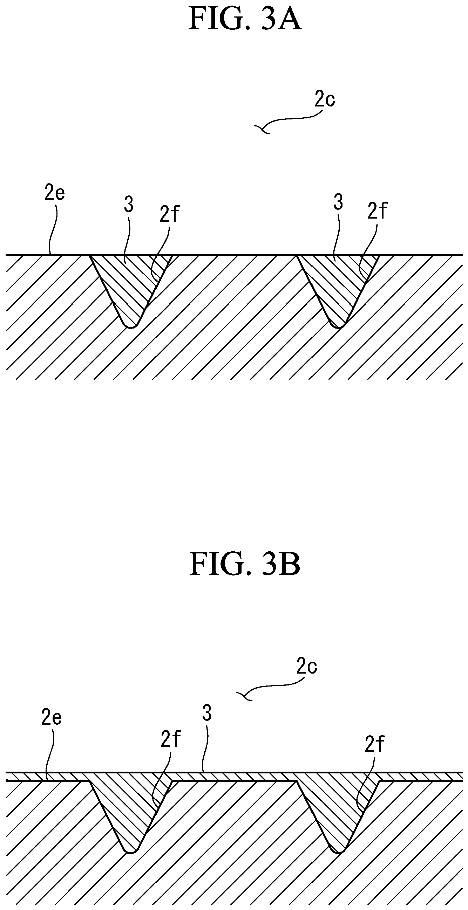

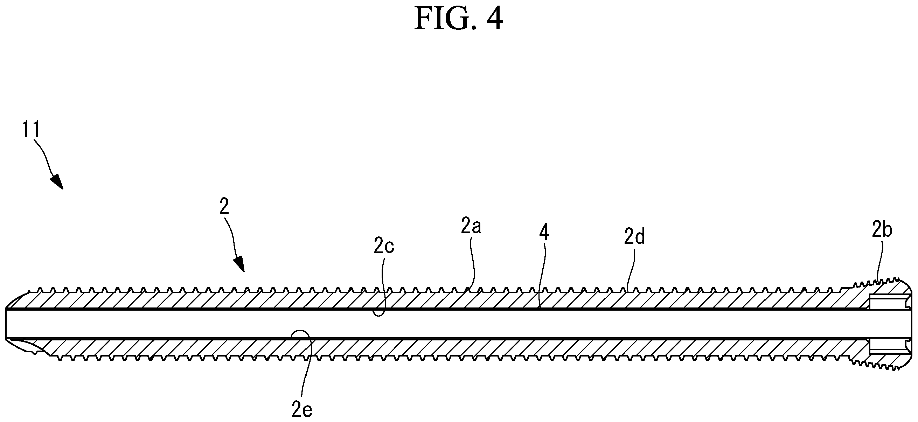

{Fig. 1} Fig. 1 is (A) a side view and (B) a plan view, as viewed from a head portion side, of a bone screw, which is an implant according to an embodiment of the present invention.
{Fig. 2} Fig. 2 is a longitudinal sectional view of the bone screw in Fig. 1.
{Fig. 3A} Fig. 3A is a diagram showing an example of an antibacterial layer formed in a recessed portion on an inner surface of a hollow portion.
{Fig. 3B} Fig. 3B is a diagram showing another example of the antibacterial layer formed in the recessed portion on the inner surface of the hollow portion.
{Fig. 4} Fig. 4 is a longitudinal sectional view of a bone screw according to another embodiment of the present invention.

### {Description of Embodiment}

An implant according to an embodiment of the present invention will be described below with reference to the drawings.

As shown in Figs. 1 and 2, an implant 1 according to this embodiment is a bone screw and includes: a hollow screw body (implant body) 2 to be screwed into a bone (biological tissue); and an antibacterial property-imparting means 3 that imparts antibacterial properties to the screw body 2.

The screw body 2 has: a shaft portion 2a that extends along a longitudinal axis A; a head portion 2b that is connected to the base end of the shaft portion 2a; and a hollow portion 2c that is a hole penetrating the screw body 2 in a direction along the longitudinal axis A.

A male thread for fixing the screw body 2 to a bone is provided on an outer circumferential surface of the shaft portion 2a, and a male thread for fixing the screw body 2 to a bone plate, which will be described later, is provided on an outer circumferential surface of the head portion 2b. A guide pin for guiding the screw body 2 when the screw body 2 is screwed into the bone is inserted into the hollow portion 2c.

The screw body 2 is formed from a biocompatible material generally used for bone screws. For example, the screw body 2 is formed from a titanium alloy or a metal such as pure titanium, a synthetic resin such as PEEK (polyether ether ketone), or a ceramic.

The antibacterial property-imparting means 3 is an antibacterial layer that is formed by means of antibacterial treatment on a surface of the screw body 2, and that covers the surface of the screw body 2. The antibacterial layer 3 contains, as an antibacterial component exhibiting antibacterial properties in the body, for example, metal ions such as silver ions or copper ions, a ceramic such as silicon nitride, iodine, or a well-known antibacterial agent or the like. The antibacterial layer 3 preferably has an antibacterial activity value of 2.0 or higher. The antibacterial activity value is measured by an antibacterial test method according to JIS Z 2801. The antibacterial component may be gradually released from the antibacterial layer 3 into a space in the hollow portion 2c.

Among surfaces of the screw body 2, the antibacterial layer 3 is formed only on an inner surface 2e of the hollow portion 2c. An outer surface 2d has lower antibacterial properties as compared with the antibacterial layer 3, for example, with an antibacterial activity value of less than 2.0. For example, the biocompatible material forming the screw body 2 is exposed on the outer surface 2d.

For example, by applying antibacterial treatment, which will be described later, to the screw body 2 in a state in which the outer surface 2d is masked with a masking material, it is possible to selectively apply the antibacterial treatment onto the inner surface 2e of the hollow portion 2c to form the antibacterial layer 3. With such an antibacterial layer 3, the hollow portion 2c is provided with higher antibacterial properties as compared with the outer surface 2d. The antibacterial layer 3 is formed on at least a portion of the inner surface 2e, and is preferably formed on the entire inner surface 2e.

The antibacterial treatment is a surface treatment for adding antibacterial properties to the surface of the screw body 2 by modifying a material surface of the screw body 2. Either a dry process or a wet process may be used as the surface treatment.

Examples of the dry process include dry plating, sputtering, thermal spraying, and heat treatment. The dry plating is, for example, vacuum deposition, physical vapor deposition (PVD), or chemical vapor deposition (CVD). The heat treatment is, for example, carburizing and quenching, nitriding, soft nitriding, or induction hardening.

Examples of the wet process include wet plating and anodization. The wet plating is, for example, electroplating, electroless plating, or chemical conversion treatment.

In a preferable example, the antibacterial layer 3 is a silver layer mainly comprising silver. In addition to silver, the silver layer may further contain impurities corresponding to antibacterial treatment for forming the silver layer. In an example, the silver layer is formed by means of vapor deposition or plating.

In the case of vapor deposition, a thin filament of silver inserted into the hollow portion 2c is heated to evaporate silver, and thus, a silver layer can be formed on the inner surface 2e.

In the case of plating, a silver layer is formed on the entire surface of the screw body 2, and an unnecessary silver layer is subsequently removed. Alternatively, plating is performed in a state in which the surface of the screw body 2 excluding the inner surface 2e is masked.

The film thickness of the silver layer 3 is preferably 0.01-100 µm.

As a result of the guide pin coming into contact with the silver layer 3 on the inner surface 2e, the silver layer 3 may be peeled off from the inner surface 2e. There is a possibility that peeled pieces of the silver layer 3 peeled off from the inner surface 2e may move to outside of the hollow portion 2c, and thus affect cells around the screw body 2. Therefore, it is preferable that the peeling amount of the silver layer 3 be small. By limiting the film thickness to 100 µm or less, even if the silver layer 3 is peeled off, it is possible to suppress the peeling amount.

In addition, in order to ensure sufficient antibacterial properties of the silver layer 3, the film thickness is preferably 0.01 µm or more. It is technically difficult to control the film thickness to the order of less than 0.01 µm.

In view of suppressing the peeling amount of the silver layer 3 and having high antibacterial properties, the film thickness is more preferably 0.1-10 µm. In order to further suppress the peeling amount of the silver layer 3, the film thickness may be set to 1 µm or less.

Next, the operation of the bone screw 1 will be described.

In an example use of the bone screw 1, the bone screw 1 is used to fix a bone plate disposed at a fracture site in a patient to a bone. The bone plate has a female thread to be fastened to the head portion 2b.

First, the guide pin is inserted into a bone along a path in which the screw body 2 is to be screwed, and the bone plate is disposed on the surface of the bone such that the guide pin penetrates through the female thread. Next, the shaft portion 2a of the screw body 2 is screwed into the bone along the guide pin inserted into the hollow portion 2c. Then, the bone plate is fixed to the bone by fastening the head portion 2b to the female thread of the bone plate.

In the state in which the screw body 2 is screwed into the bone, a space that communicates with outside of the body of the patient is formed in the interior of the bone by means of the hollow portion 2c. In other words, the hollow portion 2c can be one of the main infection paths. In addition, because a tool such as the guide pin is inserted into the hollow portion 2c during surgery, bacteria are easily transferred from the tool into the air inside the hollow portion 2c and to the inner surface 2e thereof. Therefore, in the case of the hollow bone screw 1, it is important to prevent infection via the hollow portion 2c.

With this embodiment, antibacterial properties are imparted to the hollow portion 2c by means of the antibacterial layer 3 covering the inner surface 2e. Thus, it is possible to effectively prevent infection that may occur during surgery.

In addition, in view of the influence on bone fusion between the screw body 2 and a bone and the influence on cells around the screw body 2, it is difficult to provide an antibacterial layer having high antibacterial properties on the outer surface 2d of the screw body 2 that comes into direct contact with the bone. In contrast, because the hollow portion 2c does not come into direct contact with the bone, the antibacterial properties of the antibacterial layer 3 can be enhanced without increasing the influence on the bone fusion and the cells. For example, an antibacterial layer 3 containing a strong antibacterial component or an antibacterial layer 3 having a high concentration of an antibacterial component can be provided on the inner surface 2e of the hollow portion 2c, while suppressing the influence on the bone fusion and the cells. With such an antibacterial layer 3 having high antibacterial properties, it is possible to more effectively prevent infection.

In addition, if the outer surface 2d is covered with an antibacterial layer, as described above, there is a possibility that the antibacterial properties of the antibacterial layer on the outer surface 2d may affect the bone fusion and the cells. With this embodiment, the outer surface 2d of the screw body 2 that is exposed to the bone has low antibacterial properties or has no antibacterial properties. Therefore, it is possible to effectively suppress infection while suppressing the influence on the bone fusion and the bone.

Furthermore, the outer surface 2d is rubbed against the bone in the process of screwing the screw body 2 into the bone; thus, the antibacterial layer on the outer surface 2d is easily peeled off. In contrast, because the inner surface 2e of the hollow portion 2c does not come into contact with the bone, peeling of the antibacterial layer 3 is less likely to occur; thus, the antibacterial layer 3 continues to be present on the inner surface 2e even after the screw body 2 is screwed into the bone. Therefore, after the screw body 2 is screwed into the bone, the antibacterial properties of the hollow portion 2c can be reliably exhibited by means of the antibacterial layer 3.

In addition, after the screw body 2 is screwed into the bone, the antibacterial component contained in the antibacterial layer 3 is carried to an area surrounding the screw body 2 by a bodily fluid, such as blood, moving between the hollow portion 2c and the bone. Thus, suppression of infection can be expected not only in the hollow portion 2c but also in the area outside the screw body 2.

In this embodiment, the inner diameter of the hollow portion 2c at a distal end side may be larger than the inner diameter of the hollow portion 2c at the base end side. For example, the inner diameter of the hollow portion 2c may gradually increase from the base end side toward the distal end side. The difference between the inner diameter at the distal end side and the inner diameter at the base end side is preferably equal to or more than 0.01 and equal to or less than 1 mm.

With this configuration, it is possible to reduce contact between the guide pin and the antibacterial layer 3 on the inner surface 2e at the distal end side of the hollow portion 2c, thereby further reducing the peeling amount of the antibacterial layer 3.

In this embodiment, the film thickness of the antibacterial layer 3 may be uniform or non-uniform. For example, in order to suppress the peeling amount of the antibacterial layer 3 at the distal end side of the hollow portion 2c, the film thickness may gradually decrease from the base end side toward the distal end side.

In addition, the antibacterial layer 3 may be formed only on a portion of the inner surface 2e. In the case in which the antibacterial layer 3 is formed only on a portion of the inner surface 2e, the antibacterial layer 3 may be intermittently distributed along the length of the hollow portion 2c to obtain the antibacterial properties in the entire length of the hollow portion 2c. For example, the antibacterial layer 3 may be formed only on a portion in the circumferential direction of a smooth cylindrical inner surface 2e.

As shown in Fig. 3A, the inner surface 2e of the hollow portion 2c may have a recessed portion 2f that is recessed outward in the radial direction of the screw body 2, and the antibacterial layer 3 may be formed only in the recessed portion 2f. For example, the recessed portion 2f is a groove having a spiral, screw-like, or linear shape. The depth of the recessed portion 2f is preferably equal to or more than 0.1 to equal to or less than 1 mm. Because the antibacterial layer 3 in the recessed portion 2f is unlikely to come into contact with the guide pin inserted into the hollow portion 2c, it is possible to prevent peeling of the antibacterial layer 3.

As shown in Fig. 3B, the antibacterial layer 3 may also be formed on the inner surface 2e outside the recessed portion 2f. In this case, the antibacterial layer 3 inside the recessed portion 2f is thick, and the antibacterial layer 3 outside the recessed portion 2f is thin.

The hollow portion 2c is also capable of receiving a liquid injected from one of openings at the head portion 2b side and the distal end side of the shaft portion 2a, and capable of discharging the liquid that has passed through the hollow portion 2c from the other opening. In addition, it is permissible to employ a configuration in which a hole or a slit for communicating between the inner circumferential surface of the hollow portion 2c and the outer circumferential surface of the shaft portion 2a is provided, so that the injected liquid is discharged also from the outer circumferential surface of the shaft portion 2a.

The hollow portion 2c of the bone screw 1 indwelled in a bone may be percutaneously injected with an antibacterial drug solution, a growth factor promoting osteogenesis, a self-tissue-derived component (PRP, bone marrow), a bone cement, or the like. Such postoperative percutaneous liquid injection may be performed multiple times, and there is a possibility that new bacteria may enter the hollow portion 2c. With this embodiment, it is possible to prevent proliferation of bacteria and infection by means of the antibacterial layer 3 on the inner surface 2e of the hollow portion 2c.

It is preferable that a distal end portion of an injection tool for injecting a liquid into the hollow portion 2c have a structure capable of sealing an inlet of the hollow portion 2c so that the liquid does not leak from the inlet of the hollow portion 2c. For example, the distal end portion of the injection tool may have a structure capable of being in close contact with the inlet of the hollow portion 2c by means of a thread portion formed in the interior of the hollow portion 2c, a thread portion of a component disposed inside the hollow portion 2c, or a compression force other than a thread structure. In order to reduce time and effort during postoperative injection, the distal end portion of the injection tool may be an indwelling needle or an indwelling pipe. In addition, it is desirable that the injection tool be subjected to antibacterial treatment in the same manner as the bone screw 1.

Although the antibacterial property-imparting means is the antibacterial layer 3 in this embodiment, alternatively, another means may be employed.

Fig. 4 shows a bone screw 11 according to another embodiment of the present invention. The bone screw 11 includes, as the antibacterial property-imparting means, an antibacterial member 4 that has antibacterial properties and that is inserted into the hollow portion 2c. For example, the antibacterial member 4 is a member having a surface covered with an antibacterial layer containing an antibacterial component. The bone screw 11 may include the antibacterial layer 3 in addition to the antibacterial member 4.

The antibacterial member 4 in Fig. 4 is an elongated columnar member that is disposed over substantially the entire length of the hollow portion 2c in the longitudinal direction, and that seals substantially the entirety of the hollow portion 2c. The antibacterial member 4 may be a lid-like member that is disposed only at an end portion of the hollow portion 2c on the head portion 2b side, and that seals only the end portion of the hollow portion 2c.

By inserting the antibacterial member 4 into the hollow portion 2c, it is possible to block communication between the hollow portion 2c and outside of the body, which causes infection, and also to impart antibacterial properties to the hollow portion 2c with a simple operation. In addition, as the screw body 2, it is possible to use a screw in which the inner surface 2e is not subjected to antibacterial treatment or the inner surface 2e has low antibacterial properties.

Although the antibacterial layer is not formed on the outer surface 2d of the screw body 2 in this embodiment, an antibacterial layer may be formed on the outer surface 2d so long as the hollow portion 2c has higher antibacterial properties as compared with the outer surface 2d. In other words, an antibacterial layer having lower antibacterial properties than the antibacterial layer of the hollow portion 2c may be formed on a portion or the whole of the outer surface 2d.

Although the bone screws 1, 11 have been described in the abovementioned embodiment, the antibacterial property-imparting means 3, 4 in this embodiment can be applied to other types of orthopedic implants having a hollow portion. In other words, the implant of the present invention is not limited to the bone screw, and encompasses other types of orthopedic implants having a hollow portion. For example, the implant may be a hollow pin to be inserted into a bone or other biological tissues, or a threaded hollow pin in which a portion thereof is a male thread.

### {Reference Signs List}

1, 11 bone screw, implant
2 screw body, implant body
2a shaft portion
2b head portion
2c hollow portion
2d outer surface
2e inner surface
2f recessed portion
3 silver layer, antibacterial layer, antibacterial property-imparting means
4 antibacterial member, antibacterial property-imparting means

## Claims

1. An implant comprising:
an implant body to be inserted into biological tissue, the implant body having a hollow portion that penetrates the implant body; and
an antibacterial property-imparting means that imparts an antibacterial property to at least the hollow portion of the implant body.

2. An implant according to claim 1, wherein the antibacterial property-imparting means imparts a higher level of the antibacterial property to the hollow portion as compared with an outer surface of the implant body.

3. An implant according to claim 2, wherein the outer surface of the implant body is not subjected to antibacterial treatment.

4. An implant according to any one of claims 1 to 3, wherein the antibacterial property-imparting means is an antibacterial layer that is formed by means of antibacterial treatment on a surface of the implant body, and the antibacterial layer is formed on at least an inner surface of the hollow portion.

5. An implant according to claim 4, wherein the antibacterial layer is a silver layer.

6. An implant according to claim 5, wherein the silver layer is formed only on the inner surface of the hollow portion among the surfaces of the implant body.

7. An implant according to claim 5 or 6, wherein a film thickness of the silver layer is equal to or more than 0.1 to equal to or less than 10 µm.

8. An implant according to any one of claims 4 to 7, wherein:
the inner surface of the hollow portion has a recessed portion that is recessed toward an outside of the implant body; and
the antibacterial layer is formed in the recessed portion.

9. An implant according to any one of claims 1 to 3, wherein the antibacterial property-imparting means is an antibacterial member that is inserted into the hollow portion.

10. An implant according to any one of claims 1 to 9, wherein the implant is a bone screw including, as the implant body, a screw body that is screwed into a bone, and the hollow portion penetrates the screw body in a direction along a longitudinal axis of the screw body.
